# EUROPEAN PATENT SPECIFICATION

(11) **EP 3 732 691 B1**
(45) Date of publication and mention of the grant of the patent: **28.05.2025**
(21) Application number: 19797050.2
(22) Date of filing: 23.04.2019
(51) Int. Cl.: G16H 10/60, G16H 50/30, G16H 40/63, G16H 10/20, G16H 15/00, G16H 50/20

(54) **METHOD AND APPARATUS FOR PROVIDING PERSONALIZED HEALTHCARE ADVICE**
VERFAHREN UND VORRICHTUNG ZUR BEREITSTELLUNG PERSONALISIERTER GESUNDHEITSTIPPS
PROCÉDÉ ET APPAREIL DE FOURNITURE DE CONSEIL PERSONNALISÉ EN SOINS DE SANTÉ

(30) Priority: 02.05.2018 IN 201841016547
(43) Date of publication of application: 04.11.2020
(73) Proprietor: Samsung Electronics Co., Ltd., Gyeonggi-do 16677 (KR)
(72) Inventor: ROY, Raj, Bangalore, 560 037 (IN); ADARSH, Kumar, Bangalore, 560 037 (IN); BUSETTY, Shiva Murthy, Bangalore, 560 037 (IN)
(74) Representative: Taor, Simon Edward William
(86) International application number: PCT/KR2019/004879
(87) International publication number: WO 2019/212180

(56) References cited:
- KR-A- 20140 028 929
- KR-A- 20140 120 615
- KR-A- 20160 055 749
- KR-A- 20170 022 007
- KR-A- 20170 041 987
- US-A1- 2017 199 189
- US-B1- 7 421 285
- DAVID ANDRE ET AL: "The Development of the SenseWear armband, a Revolutionary Energy Assessment Device to Assess Physical Activity and Lifestyle", BODYMEDIA, 1 January 2006 (2006-01-01), pages 1 - 19, XP055536491, Retrieved from the Internet <URL:https://pdfs.semanticscholar.org/e9e1/15cb6f381a706687982906d45ed28d40bbac.pdf> [retrieved on 20181219]

## Description

### Technical Field

The disclosure relates to healthcare. More particularly, the disclosure relates to a method and electronic device for providing personalized health-related advice.

### Background Art

Health is a matter of huge concern to people throughout the world. Some of the challenges with respect to global health include access to comprehensive and quality health care services, which is important for promoting and maintaining health, preventing and managing disease, and reducing unnecessary disability and premature death.

The above information is presented as background information only to assist with an understanding of the disclosure. No determination has been made, and no assertion is made, as to whether any of the above might be applicable as prior art with regard to the disclosure.

US 7 421 285, David Andre ET AL: "The Development of the SenseWear armband, a Revolutionary Energy Assessment Device to Assess Physical Activity and Lifestyle",BodyMedia, 1 January 2006 (2006-01-01), pages 1-19 and US 2017/199189 all address similar devices, but none of them mentions the reception and the accounting for the missing data due to inactivity of the device.

### Disclosure of Invention

### Technical Solution

In accordance with an aspect of the disclosure, a method and electronic device for providing personalized health-related advice is provided. According to an aspect, there is provided a method as set out in claim 1. Optional features are set out in claims 2 to 12.

According to an aspect, there is provided an apparatus as set out in claim 13.

According to an aspect, there is provided a non-transitory computer-readable medium as set out in claim 14.

### Advantageous Effects of Invention

The disclosure enables a user of an electronic device to obtain medical advice from the electronic device based on the symptom input, medical record stored in the electronic device or a database of a server connectable to the electronic device, and prior activities of the user.

### Brief Description of Drawings

The above and other aspects, features, and advantages of certain embodiments of the disclosure will be more apparent from the following description taken in conjunction with the accompanying drawings, in which:
FIG. 1 is a block diagram illustrating various hardware elements of an electronic device for providing personalized health-related advice, according to an embodiment of the disclosure;
FIG. 2A is a flowchart illustrating a method of providing the personalized health-related advice by the electronic device, according to an embodiment of the disclosure;
FIG. 2B is a flowchart illustrating a method of providing the personalized health-related advice by the electronic device, based on one or more health indicators, according to an embodiment of the disclosure;
FIG. 2C is a flowchart illustrating a method of providing the personalized health-related advice by the electronic device, based on one or more relevant symptoms received from the user, according to an embodiment of the disclosure;
FIG. 2D is a flowchart illustrating a method of generating a report and sharing it with a caregiver of the user, according to an embodiment of the disclosure;
FIGS. 3A and 3B are example illustrations in which the electronic device provides the personalized health-related advice to the user, based on medical record information and activities performed by the user, according to various embodiments of the disclosure;
FIG. 3C is an example scenario, in which the electronic device of the caregiver of the user receives a health summary report of the user, according to an embodiment of the disclosure;
FIGS. 4A, 4B and 4C are example illustrations in which the electronic device provides the personalized health-related advice to the user by relating a chronic condition of the user and user activities, according to various embodiments of the disclosure;
FIG. 4D is an example illustration in which an electronic device of the caregiver of the user accesses the health summary report of the user, according to an embodiment of the disclosure;
FIGS. 5A, 5B, 5C, 5D, 5E, 5F and 5G are example illustrations in which the electronic device provides the personalized health-related advice to the user, based on a real time vital parameter and the activities performed by the user, according to an embodiment of the disclosure;
FIGS. 6A, 6B and 6C are example illustrations in which the electronic device provides the health summary report and information related to health indicators of the user to the caregiver, according to various embodiments of the disclosure;
FIGS. 7A, 7B, and 7C are example scenarios illustrating implicit symptom context detection for identifying lifestyle related activities associated with the symptoms received from the user, according to various embodiments of the disclosure;
FIGS. 8A, 8B, and 8C are example illustrations of selection of a relevant health indicator for determining the lifestyle related activities associated with the symptoms provided by the user, according to various embodiments of the disclosure; and
FIGS. 9A and 9B are example illustrations in which the electronic device acquires relevant health indicators which are not stored in a medical information database, according to various embodiments of the disclosure.

The same reference numerals are used to represent the same elements throughout the drawings.

### Best Mode for Carrying out the Invention

Aspects of the disclosure are to address at least the above-mentioned problems and/or disadvantages and to provide at least the advantages described below. Accordingly, an aspect of the disclosure is to provide a method and electronic device for providing personalized health-related advice.

Additional aspects will be set forth in part in the description which follows and, in part, will be apparent from the description, or may be learned by practice of the presented embodiments.

In accordance with an aspect of the disclosure, a method and electronic device for providing personalized health-related advice is provided. The method includes receiving, at the device, a user input indicating a symptom related to the user, retrieving, from the device, at least one medical information of the user, detecting a user activity prior to the symptom arising, correlating the user input indicating the symptom, the at least one medical information of the user and the user activity prior to the symptom arising, and displaying advice about the symptom based on the correlation.

In accordance with another aspect of the disclosure, the method includes detecting the user activity prior to the symptom arising either by using at least one sensor or by receiving an activity input from the user.

In accordance with another aspect of the disclosure, the detecting of the user activity prior to the symptom arising comprises detecting the user activity prior to the symptom arising by determining at least one of a movement, position in space, inspiratory time, expiratory time, elongation, motion, temperature, impact, speed, cadence, proximity, flexibility, movement, velocity, acceleration, posture, relative motion between limbs and trunk, location of the user using the at least one sensor.

In accordance with an aspect of the disclosure, the at least one sensor comprises includes at least one of a motion sensor, a global positioning system (GPS), a thermometer, a humidity meter, a physiological sensor, one or more sleep and fitness trackers, one or more blood pressure cuffs, a pulse oximeter, or an inertial sensor including at least one of an accelerometer, a gyroscope, or a magnetoscope.

In accordance with another aspect of the disclosure, the physiological sensor is configured to determine health indicators including at least one of body temperature, respiratory rate, heart rate, skin conductivity, perspiration, pulse, stress, glucose level, pH of the user, responses to transdermal activation, electrical activity of the brain, electrical activity of muscles, arterial oxygen saturation, muscle oxygenation, oxyhemoglobin concentration, deoxyhemoglobin concentration, cardiac pacing, or cardiac rhythm of the user.

In accordance with another aspect of the disclosure, the method further includes health indicators of the user, and wherein the correlating includes correlating the user input, the at least one of medical information of the user, the user activity prior to the symptom arising, and the health indicators of the user.

In accordance with another aspect of the disclosure, the method further includes, in response to the user input indicating the symptom related to the user, displaying a query about one or more symptoms or at least one health indicator to the user, receiving a second user input indicating the one or more symptoms or the at least one health indicator from the user, the second user input being composed of a natural language, and analyzing the at least one health indicator, the user activity, the at least one medical information, or the symptom, wherein the correlating is performed based on the analyzing.

In accordance with another aspect of the disclosure, the method further includes storing the symptom, the at least one health indicators and the user activity in a database of the device or in a database of a server.

In accordance with another aspect of the disclosure, the method further includes identifying lifestyle parameters of the user, wherein the correlating includes correlating the user input indicating the symptom, the at least one of medical information of the user, the user activity prior to the symptom arising, or the lifestyle parameters of the user, and the method further includes identifying a cause of the symptom based on the correlation, and wherein the advice comprises the cause of the symptom.

In accordance with another aspect of the disclosure, the method further includes identifying the symptom based on parsing the user input using a natural language processing, wherein the user input is composed of a natural language.

In accordance with another aspect of the disclosure, the advice comprises healthcare suggestions including a suggestion of a possible lifestyle related activity to mitigate the symptom or a suggestion of transmitting an alert to a caregiver of the user.

In accordance with another aspect of the disclosure, the method further includes identifying the user activity as a cause of the symptom based on an analysis with and the correlating of the user input, the at least one medical information of the user, or the user activity prior to the symptom arising, wherein the displaying of the advice comprises displaying the advice including the cause of the symptom.

In accordance with another aspect of the disclosure, the method further includes generating an electronic report including the symptoms and the cause of the symptom, and transmitting the electronic report to a device of a caregiver of the user.

In accordance with another aspect of the disclosure, the method further includes generating a data item representing at least one health indicators of the user, the data item launched in one or more health applications, associating the symptom with the at least one health indicators, wherein the at least one health indicators is determined by an input on the data item by the user, identifying at least one secondary symptom relevant to the at least one health indicators based on the association, and updating the electronic report to include the at least one secondary symptom and a secondary cause of the at least one secondary symptom.

In accordance with another aspect of the disclosure, the at least one medical information of the user comprises at least one of information input by the user, chronic condition of the user, information from medical reports of the user, consultation history, patient medication, information from one or more health applications of the user, or health indication parameters of the user.

In accordance with another aspect of the disclosure, an electronic device for providing personalized health-related advice is provided. The device includes at least one processor configured to control a user interface to receive a user input indicating a symptom related to the user, retrieve from a memory at least one medical information of the user, detect a user activity prior to the symptom arising, correlate the user input indicating the symptom, the at least one medical information of the user and the user activity prior to the symptom arising, and a display for displaying advice about the symptom based on the correlation.

In accordance with another aspect of the disclosure, an electronic device for providing personalized health-related advice is provided. The electronic device includes a memory, at least one processor connected to the memory, a virtual assistant and a symptom analyzer. The virtual assistant is configured to receive a user input indicating a symptom related to the user. The symptom analyzer is configured to retrieve at least one of medical record information of the user, a user activity prior to the symptom arising, or lifestyle related parameters of the user. The symptom analyzer is further configured to correlate the user input and the at least one of the medical record information of the user, the user activity, or the lifestyle related parameters of the user and identify a lifestyle related activity associated with the symptom, based on the correlation. The symptom analyzer is further configured to present to the user the identified lifestyle related activity associated with the symptom as the cause of the symptom.

These and other aspects of the embodiments disclosed herein will be better appreciated and understood when considered in conjunction with the following description and the accompanying drawings. It should be understood, however, that the following descriptions, while indicating example embodiments and numerous specific details thereof, are given by way of illustration and not of limitation. Many changes and modifications may be made within the scope of the disclosure as defined by the appended claims

Other aspects, advantages, and salient features of the disclosure will become apparent to those skilled in the art from the following detailed description, which, taken in conjunction with the annexed drawings, discloses various embodiments of the disclosure.

### Mode for the Invention

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope of the disclosure as defined by the appended claims. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure , which is only defined by the appended claims.

It is to be understood that the singular forms "a," "an," and "the" include plural referents unless the context clearly dictates otherwise. Thus, for example, reference to "a component surface" includes reference to one or more of such surfaces.

Also, the various embodiments described herein are not necessarily mutually exclusive, as some embodiments can be combined with one or more other embodiments to form new embodiments. Herein, the term "or" as used herein, refers to a non-exclusive or, unless otherwise indicated. The examples used herein are intended merely to facilitate an understanding of ways in which the embodiments herein can be practiced and to further enable those skilled in the art to practice the embodiments herein. Accordingly, the examples should not be construed as limiting the scope of the embodiments herein.

As is traditional in the field, embodiments may be described and illustrated in terms of blocks which carry out a described function or functions. These blocks, which may be referred to herein as managers, engines, controllers, units or modules or the like, are physically implemented by analog and/or digital circuits such as logic gates, integrated circuits, microprocessors, microcontrollers, memory circuits, passive electronic components, active electronic components, optical components, hardwired circuits, and the like, and may optionally be driven by firmware and software. The circuits may, for example, be embodied in one or more semiconductor chips, or on substrate supports such as printed circuit boards and the like. The circuits constituting a block may be implemented by dedicated hardware, or by a processor (e.g., one or more programmed microprocessors and associated circuitry), or by a combination of dedicated hardware to perform some functions of the block and a processor to perform other functions of the block. Each block of the embodiments may be physically separated into two or more interacting and discrete blocks without departing from the scope of the disclosure. Likewise, the blocks of the embodiments may be physically combined into more complex blocks without departing from the scope of the disclosure.

In the current age of information and technology, users of electronic devices often conduct prior searches regarding symptoms they are experiencing before approaching a qualified medical professional. Furthermore, a major cause of concern is a possible wrong presumption by a user about his or her health condition based on certain symptoms experienced by the user due to generalized information obtained from public forums. When the user queries a symptom in the electronic device, the response is derived by matching multiple keywords to data or documents. The query may not retrieve information about the symptom which is relevant to the user, thereby requiring inference by the user. Also, when the query entered by the user is related to the symptom the inference by the user may lead to unintended consequences, leading to a panic situation for the user/patient and caregivers. Furthermore, conventional systems like clinical decision support systems (CDSS) and telemonitoring systems provide diagnosis and review of the diagnosis by doctors with a focus on identification of diseases based on symptoms provided by the patient.

Therefore, searching for data on the electronic device based on the symptoms provided by the user may not always result in relevant information which helps the user. Also, the conventional systems generally focus on the identification of diseases based on the symptoms provided by the user. Furthermore, the user may suspect a pre-existing chronic illness to be the primary cause of any symptom experienced without taking into consideration other factors like recent activities performed by the user, which may be the actual cause of the symptom experienced by the user.

For example, in a scenario where a user, who suffers from a chronic condition of asthma, experiences breathlessness, the user may wrongfully assume that the breathlessness is due to the asthma. However, the real cause for the breathlessness experienced by the user may be due to a heavy meal consumed by the user sometime before experiencing the breathlessness and thus, may be due to a user activity of the user prior to the symptom arising.

Accordingly, the embodiments disclosed herein provide a method and electronic device for providing personalized health-related advice. The method includes receiving a user input indicating a symptom related to the user and retrieving at least one of medical record information of the user, a user activity prior to the symptom arising, and lifestyle related parameters of the user. The method further includes correlating the user input and the at least one of the medical record information of the user, the user activity, and the lifestyle related parameters of the user and identifying a lifestyle related activity associated with the symptom, based on the correlation. The method further includes displaying to the user an advice based on the identified lifestyle related activity associated with the symptom as a cause of the symptom.

In an embodiment, the user input is at least one of a text input, a voice input, and a gesture input.

In an embodiment, the retrieving of the medical record information of the user comprises retrieving health related information from at least one of emails, reports, messages, conversations with experts, sensor data, and health records received from the user.

In an embodiment, the retrieving of the user activity comprises receiving information related to the activity of the user from at least one of the sensor data and information received from the user input.

In an embodiment, the receiving of the user input indicating the symptom related to the user includes identifying whether the user input and the at least one of the medical record information of the user, the user activity, and the lifestyle related parameters of the user are insufficient to identify the lifestyle related activity associated with the symptom. The method further includes, when it is determined that the user input is insufficient to identify the lifestyle related activity associated with the symptom, providing one or more symptoms and health indicators, where the one or more symptoms and the health indicators are related to the user input. The method further includes receiving an input from the user with respect to the one or more symptoms and the health indictors and correlating the user input and the at least one of the medical record information of the user, the user activity, and the lifestyle related parameters of the user to provide one or more causes of the one or more symptoms.

In an embodiment, the method further includes associating the one or more symptoms with a chronic condition of the user and presenting the one or more symptoms associated with the chronic condition to the user.

In an embodiment, the method further includes recommending a healthcare suggestion and/or an advice related to the symptom to the user.

In an embodiment, the method further includes generating a report including symptoms and one or more causes of the symptoms, based on a response from the user and sending the generated report to a caregiver of the user.

In an embodiment, the method includes generating a data item representing one or more health indicators of the user, where the data item is launched in one or more health applications based on an input from the user and associating the symptoms with the one or more health indicators. The method further includes providing one or more secondary symptoms to the user based on the association and receiving an input from the user with respect to one or more symptoms relevant to the secondary symptoms. The method further includes updating the report including the symptoms and the one or more causes of the symptoms and sending the updated report to the caregiver of the user.

In an embodiment, the data item representing one or more health indicators of the user is in the form of a widget.

In an embodiment, the report comprises the symptoms, the one or more causes of the symptoms, at least one of a current health condition, a past medical condition, a user activity causing the current health condition, data items, a healthcare suggestion provided to the user, and expert information about the health condition.

The disclosure allows automatic tracking of patient data (e.g., heart rate) and also allows a patient to manually enter patient data (e.g., diet related data). Furthermore, a health summary report includes symptoms provided by the user, health data widgets providing more information about various health indicators, and also the causes provided to the user.

The method and system are used only to provide information regarding the health state of the user and not to alter a therapy pattern. Furthermore, the proposed method and system is not a replacement of a medical practitioner like a doctor or a nurse, but rather is a panic alleviating technique especially for patients with chronic health conditions.

The method and system analyze the symptoms provided by the user and correlates the symptoms to one of the activities performed by the user and the medical record information of the user. Furthermore, the proposed method and system also generates a health summary report which may be shared with the caregiver of the user.

In the conventional methods and systems, the user inputs a symptom and the system searches a disease database which correlates the disease and the symptoms. Furthermore, the conventional system also generates a report listing the diseases which are related to the symptom provided by the user. The proposed method and system provide personalized analysis and healthcare suggestions to the user based on the symptom input by the user.

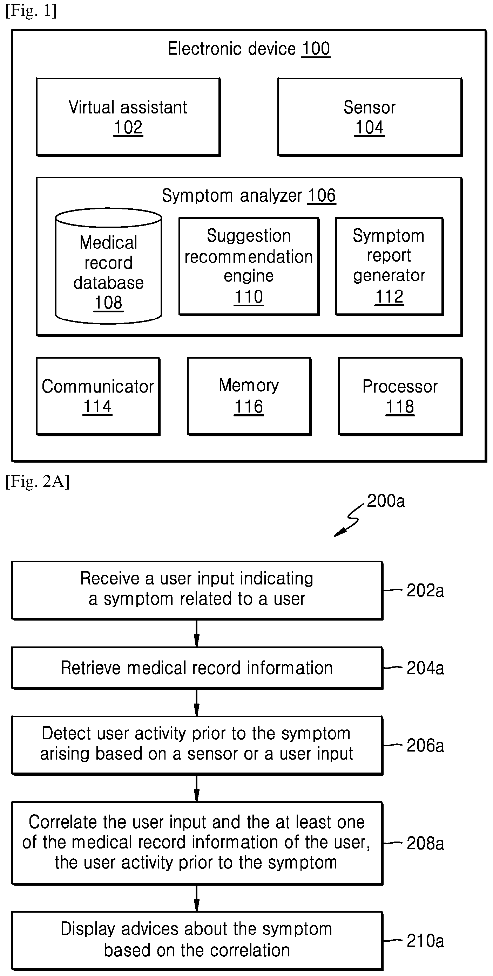

Example embodiments will now be described below by referring to the drawings, and more particularly to FIGS. 1 through 9B, wherein similar reference numerals denote similar elements throughout the figures.

FIG. 1 is a block diagram illustrating various hardware components of an electronic device 100 for providing personalized health-related advice, according to an embodiment of the disclosure.

In an embodiment, the electronic device 100 may be, for example, a mobile phone, a smart phone, a personal digital assistant (PDA), a tablet, a wearable device, a display device, an Internet of things (IoT) device, an electronic circuit, a chipset, an electrical circuit (e.g., a System on Chip (SoC)), etc.

Referring to FIG. 1, the electronic device 100 includes a virtual assistant 102, a sensor 104, a symptom analyzer 106 including a medical record database 108, a suggestion recommendation engine 110, a symptom report generator 112, a communicator 114 (e.g., a communication unit or a transceiver), a memory 116, and a processor 118 (e.g., at least one processor). The virtual assistant 102, the symptom analyzer 106, the communicator 114 and the processor 118 can be implemented as at least one hardware processor.

In an embodiment, the virtual assistant 102 is configured to receive an input indicating a symptom related to a user from the user. The input may be provided for example, in the form of at least one of a text input, a voice input and a gesture input. A user interface (UI) may be for example, a text box that allows the user to input symptoms in the form of a text message. The symptom may be a departure from normal body conditions experienced by the user such as, sweating profusely, fatigue, nausea, dizziness, breathlessness, headache, body pain, migraines, motion sickness, muscle spasm, numbness, shaking, sleep or sleep disorders, tremors, unconsciousness, allergic reactions, anxiety attacks, attention deficit hyperactivity disorders, backaches, depression, drowsiness, epileptic seizures, fatigue, heart malfunction, hunger pangs, and the like.

Furthermore, the virtual assistant 102 may also be configured to parse and recognize the input provided by the user by using pre-existing speech processing techniques. For example, the virtual assistant 102 interprets and identifies the symptom based on the recognized input by using natural language processing (NLP) and the like.

In an embodiment, the sensor 104 may include a combination of various sensors such as for example, a motion sensor, a GPS, a thermometer, a humidity meter, inertial sensors including an accelerometer, a gyroscope, and a magnetoscope, which may be configured to detect one or more user activities (i.e. , prior to the symptom experienced by the user arising). A user activity prior to the symptom experienced by the user arising may be detected by determining the movement, position in space, inspiratory time, expiratory time, elongation, motion, temperature, impact, speed, cadence, proximity, flexibility, movement, velocity, acceleration, posture, relative motion between limbs and trunk, location, etc. of the user. Furthermore, the sensor 104 may also include physiological sensors to determine health indicators of the user such as body temperature, respiratory rate, heart rate, skin conductivity, perspiration, pulse, stress, glucose level, pH, responses to transdermal activation, electrical activity of the brain, electrical activity of muscles, arterial oxygen saturation, muscle oxygenation, oxyhemoglobin concentration, deoxyhemoglobin concentration, cardiac pacing, cardiac rhythm (electrocardiogram (ECG)), and the like.

The health indicators and the user activity determined by the sensor 104 are stored in the medical record database 108 for future reference. The health indicators and the user activity may be determined either by the sensor 104 or by an input by the user. The sensor 104 may be embedded in the electronic device 100, and may include at least one of sleep and fitness trackers, blood pressure cuffs, a pulse oximeter, a physiological or biosensor worn by the user, etc.

The symptom analyzer 106 includes the medical record database 108, the suggestion recommendation engine 110, and the symptom report generator 112.

In an embodiment, the symptom analyzer 106 is configured to retrieve the medical record information of the user indicating the health of the user. The medical record information may be acquired from the user input, the sensor 104, and the medical record database 108. The medical record information of the user also includes the information about the pre-existing chronic conditions of the user. The symptom analyzer 106 also associates the chronic conditions of the user with the symptoms experienced by the user. Furthermore, the symptom analyzer 106 identifies a user activity or a lifestyle related activity associated with the symptoms provided by the user. The user activity or the lifestyle related activity associated with the symptom is identified by analyzing the symptom with respect to the user activity detected prior to the symptom, the lifestyle related indicators, and/or the medical record information of the user. The user activity may include but not limited to a workout, physical exercises, yoga, taking a walk, spending all night for certain tasks, food intake, etc. On the other hand, the lifestyle related activity may include but not limited to certain activities experienced or conducted by the user at certain regular intervals such as regular food or medicine intake, regular exercise, regular waking-up, usual sleeping time, etc.

In an embodiment, the medical record database 108 is configured to store medical record information of the user which represents the health condition of the user. The health condition of the user may be chronic conditions of the user. Furthermore, the medical record information includes at least one of information input by the user, information from medical reports of the user, consultation history, patient medication, information from one or more health applications of the user, and health indication parameters of the user previously recorded by the electronic device 100.

In an embodiment, the suggestion recommendation engine 110 is configured to analyze the symptoms provided by the user by correlating the user input, the at least one of the medical record information of the user, the user activity prior to the symptom arising. The analysis can be performed optionally with the lifestyle parameters of the user. The lifestyle parameters are parameters related to the lifestyle activity of the user such as waking-up time, sleeping time, total walking time or steps a day, usual calories or fat intake a day, usual workout time a day, etc. The symptoms provided by the user are analyzed with the health related information. The health related information includes but is not limited to various lifestyle indicators, activities performed by the user, medical record information of the user or pre-existing chronic conditions of the user.

The suggestion recommendation engine 110 is further configured to provide the cause of the symptom. The cause of the symptom is identified based on the correlation of the identified at least one of the user activity prior to the symptom, the symptom of the user and/or the medical information of the user. The lifestyle related parameters of the user can be also taken into consideration in identifying the cause of the symptom. Once the cause of the symptom is determined based on the correlation, the advice displayed on the device of the user may refer to the cause of the symptom.

The suggestion recommendation engine 110 is further configured to provide healthcare suggestions related to the symptom to the user. The healthcare suggestions may include suggesting a possible lifestyle related activity to mitigate the symptoms experienced by the user, suggesting transmitting an alert to a caregiver of the user, and the like.

The suggestion recommendation engine 110 is further configured to provide an extended symptom list to the user based on the association of the symptoms with the health indicators. The extended symptom list includes two or more symptoms which are contextually related to the symptom input by the user. Furthermore, the user is prompted to confirm if the user experiences any of the symptoms from the extended symptom list, to provide accurate analysis of the condition of the user.

In an embodiment, the symptom report generator 112 is configured to generate a health summary report with respect to the user. The health summary report with respect to the user includes the symptoms provided by the user, the medical record information and one or more causes of the symptoms. The health summary report is generated based on a response from the user i.e., only if the user wishes to generate such a health summary report, the health summary report is generated by the symptom report generator 112.

Furthermore, the symptom report generator 112 may also be configured to generate a data item representing one or more health indicators of the user. The user can access the information provided in the data item by selecting the data item. Further, the data item is launched in one or more health applications. The data item may be provided in the form of widgets. Once one or more health indicators of the user is determined based on the selection of the data item, the user's symptom is analyzed by associating the symptom with the one or more health indicators. In an embodiment, at least one secondary symptom relevant to the one or more health indicators is identified based on the associating the symptom with the one or more health indicators.

Furthermore, the symptom report generator 112 also updates the health summary report of the user based on the secondary symptoms provided by the user from the extended symptom list provided by the suggestion recommendation engine 110.

In an embodiment, the communicator 114 is configured to transmit the health summary report generated by the symptom report generator 112 to the caregiver of the user. The caregiver data is pre-stored by the user to enable the communicator to send the health summary report to the caregiver. Further, the health summary report of the user is shared with the caregiver only after obtaining the user permission.

In an embodiment, the memory 116 may include non-volatile storage elements. Examples of such non-volatile storage elements may include magnetic hard discs, optical discs, floppy discs, flash memories, or forms of electrically programmable memories (EPROM) or electrically erasable and programmable (EEPROM) memories. In addition, the memory 116 may, in some examples, be considered a non-transitory storage medium. The term "non-transitory" may indicate that the storage medium is not embodied in a carrier wave or a propagated signal. However, the term "non-transitory" should not be interpreted that the memory 116 is non-movable. In some examples, the memory 116 may be configured to store larger amounts of information than the memory. In certain examples, a non-transitory storage medium may store data that can, over time, change (e.g., in a random access memory (RAM) or cache).

In an embodiment, the processor 118 is configured to interact with the hardware elements such as the virtual assistant 102, the sensor 104, the symptom analyzer 106, the medical record database 108, the suggestion recommendation engine 110, the symptom report generator 112, the communicator 114, and the memory 116 in the electronic device 100 to provide personalized health-related advice to the user.

Although FIG. 1 shows the hardware components of the electronic device 100, it is to be understood that other embodiments are not limited thereto. In other embodiments, the electronic device 100 may include less or more number of components. Furthermore, the labels or names of the elements are used only for illustrative purpose and do not limit the scope of the disclosure. One or more components may be combined together to perform a same or substantially similar function to provide the personalized health-related advice in the electronic device 100.

FIG. 2A is a flowchart 200a illustrating the method of providing the personalized health-related advice by the electronic device 100, according to an embodiment of the disclosure.

Referring to FIG. 2A, in operation 202a, the electronic device 100 receives the user input indicating the symptom related to the user. For example, in the electronic device 100 illustrated in FIG. 1, the virtual assistant 102 is configured to receive the user input indicating the symptom related to the user.

In operation 204a, the electronic device 100 retrieves medical record information of the user. In addition, lifestyle related parameters of the user can be retrieved from the electronic device 100 as well. For example, in the electronic device 100 illustrated in FIG. 1, the symptom analyzer 106 is configured to retrieve at least one of the medical record information of the user, and the lifestyle related parameters of the user.

In operation 206a, the sensor 104 may detect the user activity prior to the symptom arising. In another embodiment, the user activity prior to the symptom arising may be determined based on a user input related to the user activity such as food intake including a kind of the food, the amount of the food, a cook type of the food, etc.

In operation 208a, the electronic device 100 correlates the user input and the at least one of the medical record information of the user, the user activity prior to the symptom. Optionally, the lifestyle related parameters of the user may be also taken into consideration in correlating. For example, in the electronic device 100 illustrated in FIG. 1, the symptom analyzer 106 is configured to correlate the user input and the at least one of the medical record information of the user, the user activity prior to the symptom arising, and/or the lifestyle related parameters of the user.

In operation 210a, the electronic device 100 may display advices about the symptom based on the correlation. The advice may include a cause of the symptom and suggest a possible lifestyle related activity to mitigate the symptom or a transmission of a notification alerting to a caregiver of the user. The electronic device 100 identifies the at least one of the user activity prior to the symptom and the lifestyle related parameters of the user based on the correlation. For example, in the electronic device 100 illustrated in FIG. 1, the symptom analyzer 106 is configured to identify a lifestyle related activity associated with the symptom, based on the correlation.

Optionally, the electronic device 100 may present the user activity as a cause of the symptom. For example, in the electronic device 100 illustrated in FIG. 1, the symptom analyzer 106 is configured to present the user activity as the cause of the symptom.

The various actions, operations, blocks, steps, or the like in the flowchart 200a may be performed in the order presented, in a different order, or simultaneously. Further, in some embodiments, some of the actions, operations, blocks, steps, or the like may be omitted, added, modified, skipped, or the like without departing from the scope of the disclosure.

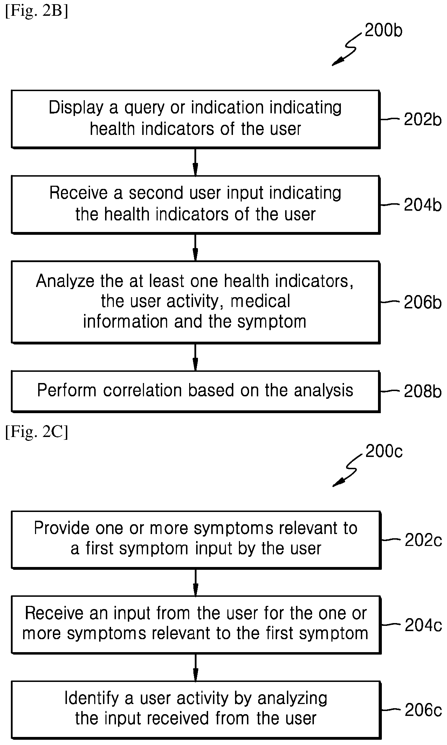

FIG. 2B is a flowchart illustrating a method of providing the personalized health-related advice by the electronic device, based on one or more health indicators, according to an embodiment of the disclosure.

Referring to FIG. 2B, in operation 202b, the electronic device 100 indicates one or more health indicators of the user related to the symptom. For example, in the electronic device 100 illustrated in FIG. 1, the virtual assistant 102 is configured to indicate one or more health indicators of the user related to the symptom. In an embodiment, the electronic device may display a query about the health indicators of the user in response to the user input indicating the symptom related to the user.

In operation 204b, the electronic device 100 receives one or more inputs from the user indicating one or more health indicators. For example, in the electronic device 100 illustrated in FIG. 1, the virtual assistant 102 is configured to receive one or more inputs from the user indicating one or more health indicators.

In operation 206b, the electronic device 100 analyzes the one or more inputs received from the user, along with the activities of the user and the medical record information, to provide one or more causes of the symptom. For example, in the electronic device 100 illustrated in FIG. 1, the symptom analyzer 106 is configured to analyze the symptom, the one or more inputs received from the user, along with the activities of the user and the medical record information, to provide the one or more causes of the symptom.

In operation 208b, the electronic device 100 may correlate the user input indicating the symptom, the at least one medical information of the user and the user activity prior to the symptom arising based on the analysis performed in operation 206b.

The various actions, operations, blocks, steps, or the like in the flowchart 200b may be performed in the order presented, in a different order, or simultaneously. Further, in some embodiments, some of the actions, operations, blocks, steps, or the like may be omitted, added, modified, skipped, or the like without departing from the scope of the disclosure.

FIG. 2C is a flowchart illustrating a method of providing the personalized health-related advice by the electronic device, based on one or more relevant symptoms received from the user, according to an embodiment of the disclosure.

Referring to FIG. 2C, in operation 202c, the electronic device 100 provides one or more symptoms relevant to the first symptom input by the user. For example, in the electronic device 100 illustrated in FIG. 1, the symptom analyzer 106 is configured to provide the one or more symptoms relevant to the symptom to the user.

In operation 204c, the electronic device 100 receives the input from the user for the one or more symptoms relevant to the symptom. For example, in the electronic device 100 illustrated in FIG. 1, the virtual assistant 102 is configured to receive the input from the user for the one or more symptoms relevant to the symptom.

In operation 206c, the electronic device 100 identifies a user activity by analyzing the input received from the user. For example, in the electronic device 100 illustrated in FIG. 1, the symptom analyzer 106 is configured to provide one or more causes of the symptom by analyzing the input received from the user.

The various actions, operations, blocks, steps, or the like in the flowchart 200c may be performed in the order presented, in a different order, or simultaneously. Further, in some embodiments, some of the actions, operations, blocks, steps, or the like may be omitted, added, modified, skipped, or the like without departing from the scope of the disclosure.

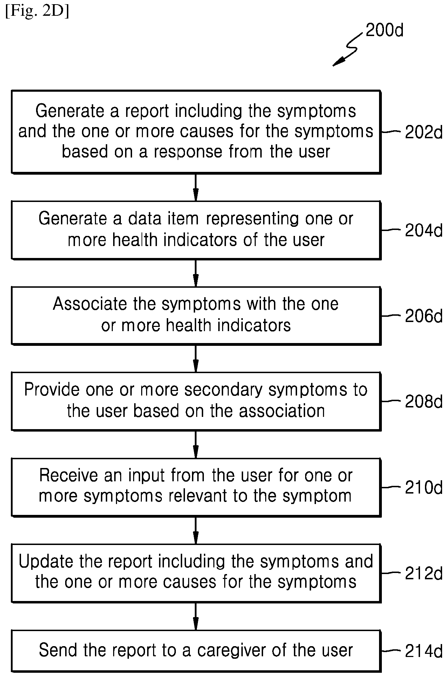

FIG. 2D is a flowchart illustrating a method of generating a report and sharing it with a caregiver of the user, according to an embodiment of the disclosure.

Referring to FIG. 2D, in operation 202d, the electronic device 100 generates a report including symptoms and one or more causes of the symptoms based on a response from the user. For example, in the electronic device 100 illustrated in FIG. 1, the symptom report generator 112 is configured to generate a report including the symptoms and the one or more causes of the symptoms based on a response from the user.

In operation 204d, the electronic device 100 generates a data item representing one or more health indicators of the user. For example, in the electronic device 100 illustrated in FIG. 1, the symptom report generator 112 is configured to generate a data item representing one or more health indicators of the user. For example, the data item can be a user interface for inputting the one or more health indicators of the user and the one or more health indicators can be determined by the user input on the data item.

In operation 206d, the electronic device 100 associates the symptoms with the one or more health indicators. For example, in the electronic device 100 illustrated in FIG. 1, the symptom analyzer 106 is configured to associate the symptoms with the one or more health indicators.

In operation 208d, the electronic device 100 provides one or more secondary symptoms to the user based on the association. For example, in the electronic device 100 illustrated in FIG. 1, the symptom report generator 112 is configured to provide one or more secondary symptoms and/or a cause of the one or more secondary symptoms to the user based on the association.

In operation 210d, the electronic device 100 receives an input from the user for one or more symptoms relevant to the symptom. For example, in the electronic device 100 illustrated in FIG. 1, the virtual assistant 102 is configured to receive an input from the user for one or more symptoms relevant to the symptom.

In operation 212d, the electronic device 100 updates the report including the symptoms and the one or more causes of the symptoms. For example, in the electronic device 100 illustrated in FIG. 1, the symptom report generator 112 is configured to update the report including the symptoms and the one or more causes of the symptoms.

In operation 214d, the electronic device 100 sends the report to a caregiver of the user. For example, in the electronic device 100 illustrated in FIG. 1, the communicator 114 is configured to send the report to the caregiver of the user.

The various actions, operations, blocks, steps, or the like in the flowchart 200d may be performed in the order presented, in a different order, or simultaneously. Further, in some embodiments, some of the actions, operations, blocks, steps, or the like may be omitted, added, modified, skipped, or the like without departing from the scope of the disclosure.

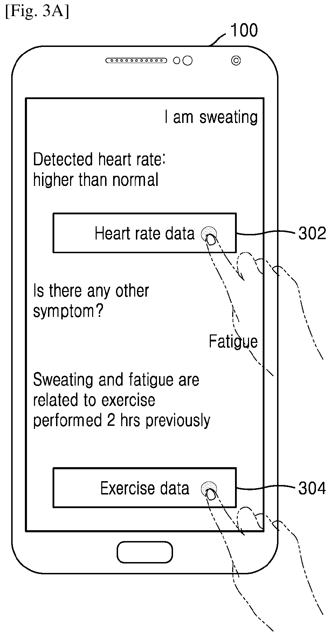

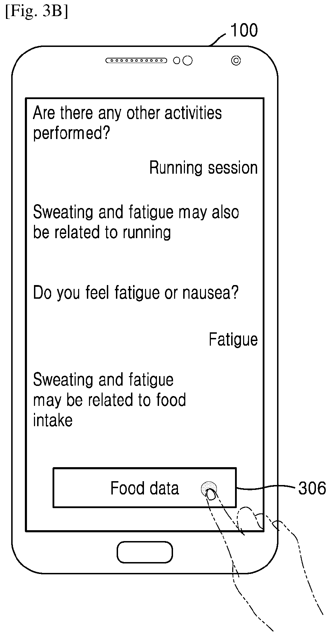

FIGS. 3A and 3B are example illustrations in which the electronic device 100 provides the personalized health-related advice to the user based on medical record information and user activities, according to various embodiments of the disclosure.

Referring to FIG. 3A, the user of the electronic device 100 provides a text or voice input such as 'I am sweating' on the UI, which indicates the symptom experienced by the user i.e., sweating. On receiving the symptom from the user, the sensor 104 (for example, a heart rate sensor in the sensor 104) of the electronic device 100 determines that the heart rate of the user as one of health indicators is higher than normal. Further, the electronic device 100 provides the heart rate data in the form of a widget 302. The user may interact with the widget to access more information related to the heart rate.

Furthermore, the electronic device 100 requests the user to provide more symptoms for providing better identification of the user activities associated with the symptom experienced by the user. In response to the requesting by the electronic device 100, the user provides more symptoms to the electronic device 100 such as 'fatigue'. The electronic device 100 analyzes the symptoms provided by the user and the sensor data (i.e., real time heart rate of the user) to identify that sweating and fatigue are associated with an activity performed by the user 2 hours previously i.e., an exercise session. Further, the details of the exercise session are provided in an exercise data widget 304.

Referring to FIG. 3B, the electronic device 100 requests the user to provide more data regarding the activities performed by the user. In response to the request, the user inputs 'running session' as the other activity performed by the user. Further, the electronic device 100 analyzes the symptoms provided by the user and the user activity to provide the cause of sweating and fatigue to be running.

Furthermore, the electronic device 100 also provides the extended list of symptoms based on the context of the symptoms provided by the user such as 'Do you feel fatigue or nausea?' to provide correct analysis of the symptoms and the causes related to the symptoms. Based on the user input and the retrieved data, the electronic device 100 then associates the symptoms with food intake of the user. The electronic device 100 also presents a diet data widget 306 which can be expanded by the user to obtain the data related to the food consumed by the user.

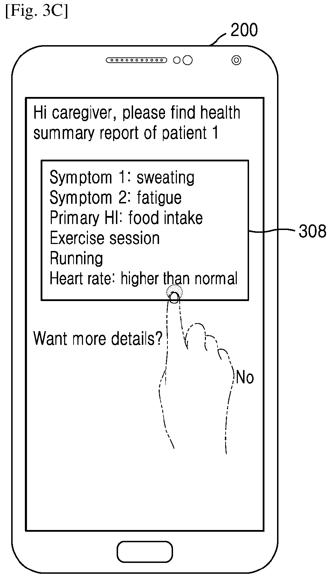

FIG. 3C is an example illustration in which the electronic device 100 generates the health summary report 308 and shares it with the caregiver of the user, according to an embodiment of the disclosure.

Referring to FIG. 3C, the electronic device 100 generates the health summary report 308 which includes the entire conversation between the user and the electronic device 100 and sends the health summary report 308 to the caregiver of the user. The health summary report 308 also includes the data widgets provided by the electronic device 100 to the user. Further, the electronic device 200 of the caregiver receives the health summary report 308, as shown in FIG. 3C.

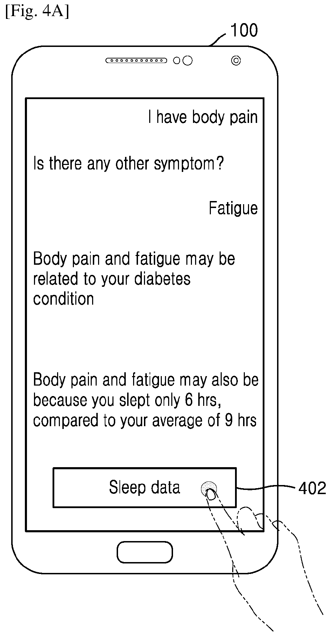

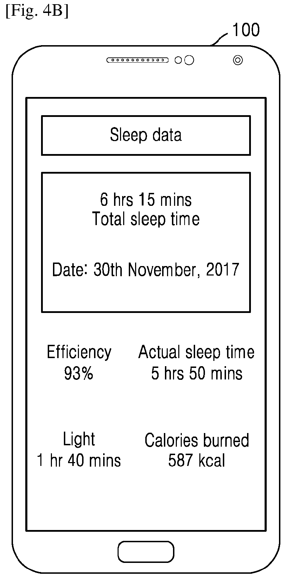

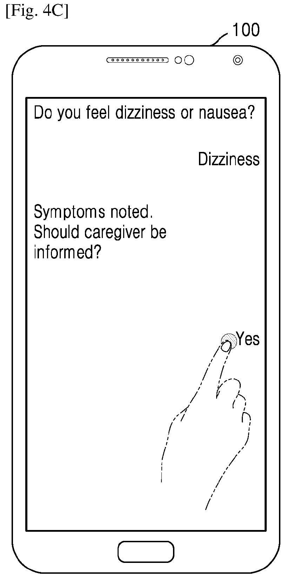

FIGS. 4A, 4B and 4C are an example illustration in which the electronic device provides personalized health-related advice to the user by relating a chronic condition of the user and activities performed by the user, according to various embodiments of the disclosure.

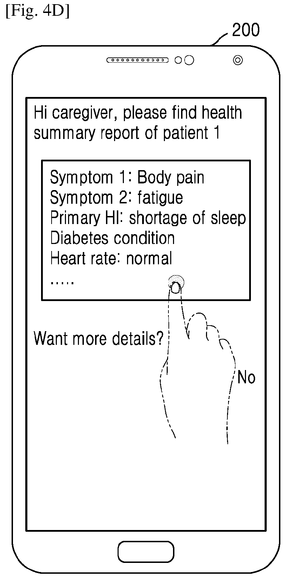

FIG. 4D is an example illustration in which an electronic device of the caregiver of the user accesses the health summary report of the user, according to an embodiment of the disclosure.

Referring to FIG. 4A, the user enters the symptoms - I have body pain - on the UI of the electronic device 100. Further, the electronic device 100 requests the user to provide more symptoms for providing better analysis of the cause of the symptom experienced by the user. In response to the requesting by the electronic device 100, the user provides more symptoms to the electronic device 100 such as 'fatigue'.

The electronic device 100 then analyzes the symptoms provided by the user and associates the symptoms with a chronic condition of the user (i.e. diabetes), which is part of the medical record information stored in the medical record database 108. Further, the electronic device 100 determines that the user's sleep pattern is not within a normal value range, i.e., the user has slept for only 6 hours as compared to an average of 9 hours (i.e., a normal value), based on the sensor 104 data. Further, the electronic device 100 provides sleep related information in a sleep data widget 402.

When the user expands the sleep data widget 402, the sleep related information is displayed due to the expanded functionality, as shown in FIG. 4B.

Referring to FIG. 4C, the electronic device 100 further provides an extended symptom list (i.e., dizziness and nausea) which are contextually relevant to the symptom already provided by the user (i.e., fatigue) and asks the user to select the symptom experienced by the user from the extended symptom list. Furthermore, the electronic device 100 requests the user's permission to share the health summary report with the caregiver of the user. On obtaining the permission from the user to share the health summary report with the caregiver, the electronic device 100 generates the health summary report and shares it with the caregiver of the user, as shown in FIG. 4D.

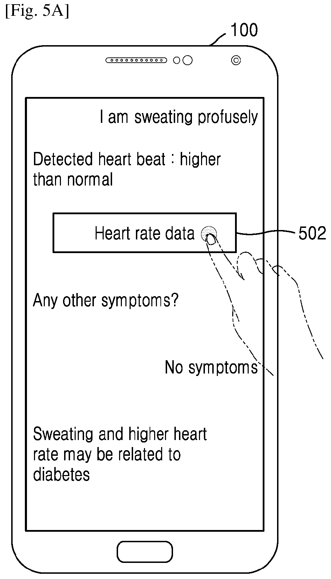

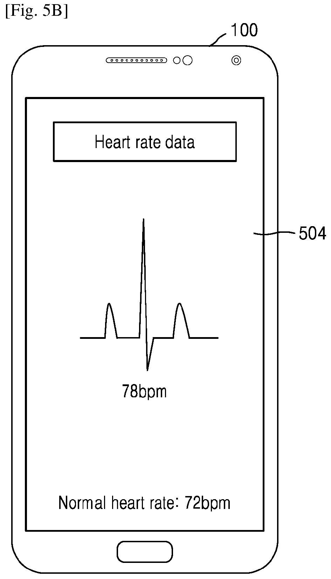

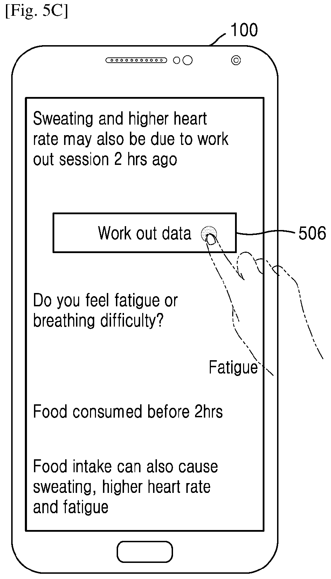

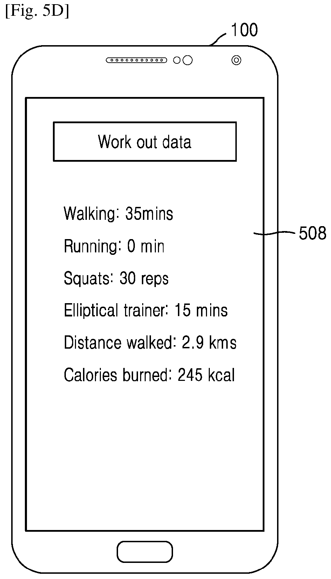

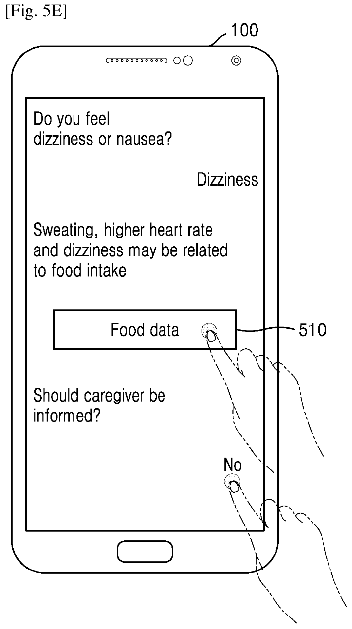

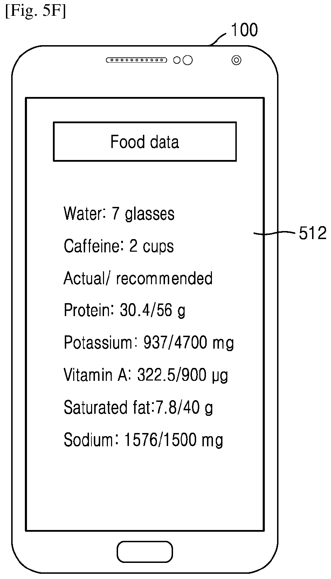

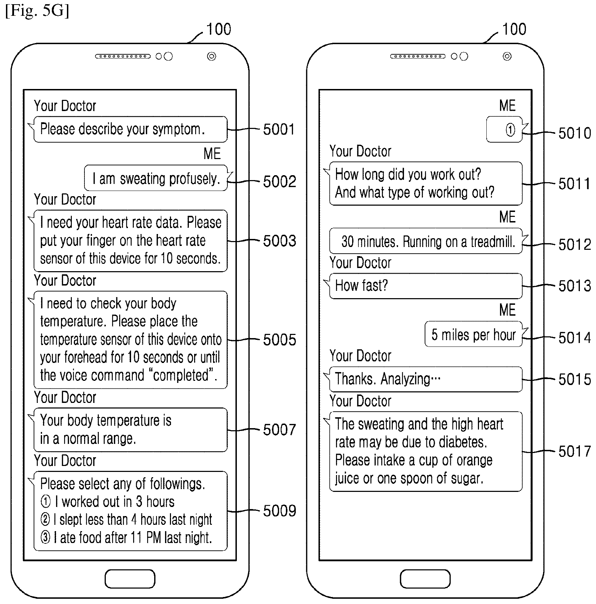

FIGS. 5A, 5B, 5C, 5D, 5E, 5F and 5G are an example illustration in which the electronic device provides the personalized health-related advice to the user, based on a real time vital parameter and activities performed by the user, according to various embodiments of the disclosure.

Referring to FIG. 5A, the user of the electronic device 100 provides an input indicating the symptom i.e., "I am sweating profusely" on the UI. On receiving the symptom from the user, the electronic device 100 may determine that the heart rate of the user is higher than normal as follows. First, the electronic device 100 may request the user to provide any biological data related to the symptom using a heart rate sensor. For example, in response to the input of "I am sweating profusely," the electronic device 100 may analyze the symptom and determine that heart rate data should be detected based on the nature of the symptom - i.e., sweating. Thus, the electronic data may display a natural language based request of "Please put your finger on the sensor."

Before the electronic device 100 determines to check the heart rate data of the user, the electronic device 100 may analyze health data of the user stored in a database of the electronic device 100 and/or connect to a server (not shown) which may be able to link any specific symptom to a related disease or any additional biological information to be extracted for an accurate determination related to the symptom inputted by the user. The server may provide to the electronic device 100 with the related disease corresponding to the symptom and/or a request for any additional biological information for an accurate analysis of the symptom.

In case that the electronic device 100 checks the health data of the user stored in the database of the electronic device 100, the electronic device 100 may be able to acknowledge the chronic condition or the chronic disease of the user based on the symptom which may be occurring from time to time. If the electronic device 100 determines that the symptom inputted by the user has no track record based on the health data stored in the database of the electronic device 100 or the server, the electronic device 100 may store or cause the server to store the symptom of the user as new symptom data related to the user.

While detecting the heart rate data using the sensor, the electronic device 100 may provide real-time heart rate data in the form of a heart rate widget 502 which provides expanded functionality related to the abnormal heart rate, as shown in FIG. 5B. Further, the expansion of the widget may provide more data in another application user interface 504, as shown in FIG. 5B. Furthermore, the electronic device 100 requests the user to provide additional symptoms. On receiving a response from the user, the electronic device 100 may associate the first symptom of the sweating and the high heart rate of the user with a chronic condition of the user, i.e., diabetes.

Referring to FIG. 5C, the electronic device 100 may analyze the symptoms provided by the user and the sensor data (i.e., heart rate data obtained from a heart rate sensor) and relates the symptoms of sweating and higher heart rate provided by the user to a work out session performed by the user 2 hours previously. The electronic device 100 may continuously record the user activity using the sensor 104 or a wearable device connected to the electronic device 100. In an embodiment, the electronic device 100 may request the user to input if he or she had any work out session within a certain time frame. For example, the electronic device 100 may ask the user - "Did you have any work out session in 3 hours from now?" Further, the electronic device 100 may provide workout related data in a workout data widget 506 using the data sensed by the sensor 104 or the input responding to the aforementioned query. The workout data widget 506 can be expanded to access the workout related information in a fitness related application 508, as shown in FIG 5D. Further, the electronic device 100 also provides more symptoms based on the context of the symptoms already provided by the user to accurately provide health related information to the user. The electronic device 100 determines that the symptoms provided by the user like sweating, higher heart rate and fatigue may also be due to food consumed by the user.

Referring to FIG. 5E, the electronic device 100 provides the extended symptom list to confirm whether the symptoms provided by the user like sweating, higher heart rate and fatigue are related to the food consumed by the user. In the extended symptom list the electronic device 100 provides more symptoms to the user and asks the user if any of the symptoms in the extended symptom list are experienced by the user. As the user provides the symptom as dizziness, the electronic device 100 provides diet related data of the user. Further, the diet related data is provided in the form of a diet data widget 510 and the diet data widget 510 can be expanded to access additional data 512 related to the food consumed by the user such as the calories, various vitamins, minerals, etc., as shown in FIG. 5F. Furthermore, the electronic device 100 relates that the symptoms experienced by the user such as the sweating, the higher heart rate and the dizziness to be related to the food consumed by the user.

FIG. 5G is an example illustration of interactive chat messages in which the electronic device provides the personalized health-related advice to the user, according to an embodiment of the disclosure.

Referring to FIG. 5G, the electronic device 100 may provide the personal health-related advice to the user in a chat message format. The electronic device 100 may analyze the queries 5001, 5009, 5011, and 5013 presented to the user and replies 5002, 5010, 5012, and 5014 by the user, the electronic device 100 may narrow down the advice to be presented to the user. If the electronic device 100 needs to check any biological data using sensors, the electronic device 100 may request the user to follow the instruction for sensing the biological data according to the chat messages 5003 and 5005. In an embodiment, the electronic device 100 may request a selection of options presented to the user for accurate determinations regarding the symptom as shown in the chat message 5009. The electronic device 100 may analyze the option selected by the user and may present additional queries if the electronic device 100 determines that additional information is/are required as shown in chat messages 5011 and 5013. Based on the chat messages and information exchanged therebetween, the electronic device 100 may determine the cause of the symptom and provide an advice according to the analysis result of the exchanged information as shown in the chat message 5017.

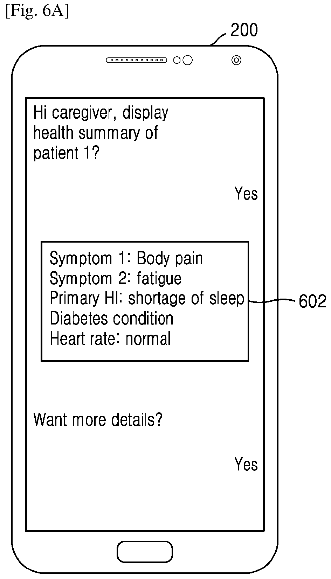

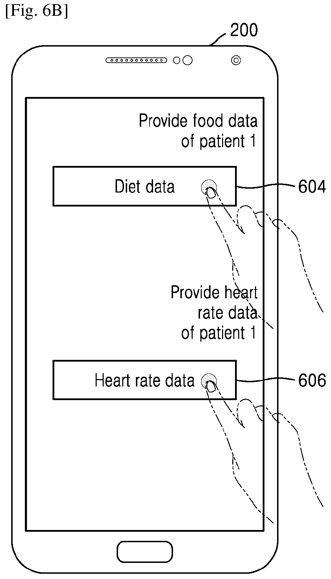

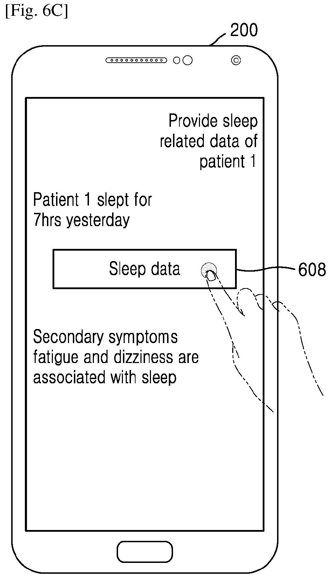

FIGS. 6A, 6B, and 6C illustrate example scenarios of the method of providing the health summary report and information related to health indicators of the user to the caregiver, according to various embodiments of the disclosure.

Referring to FIG. 6A, the caregiver requests the electronic device 200 to provide the health summary report 602 of a patient 1. The electronic device 200 displays the health summary report 602 of the patient 1, in the form of a widget. Further, the electronic device 200 interacts with the caregiver and asks whether the caregiver requires more information regarding the health of the patient 1. In response to determining that the caregiver requires more information regarding the health of the patient 1, the electronic device 200 provides information related to the activities performed by the patient 1, the health indicators, etc. The electronic device 200 provides the diet related data in the form of a diet data widget 604 and the heart rate data in the form of a heart rate data widget 606, as shown in FIG. 6B.

Further, the caregiver requests the electronic device 200 to provide sleep related information of the patient 1. In response to the caregiver's request to provide the sleep related information of the patient 1, the electronic device 200 provides sleep related data in the form of a sleep data widget 608, as shown in FIG. 6C. Further, the electronic device 200 informs the caregiver of the condition of the patient 1 by providing causes of the symptoms input by the patient 1 such as, 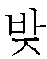 econdary symptoms fatigue and dizziness are associated with sleep."

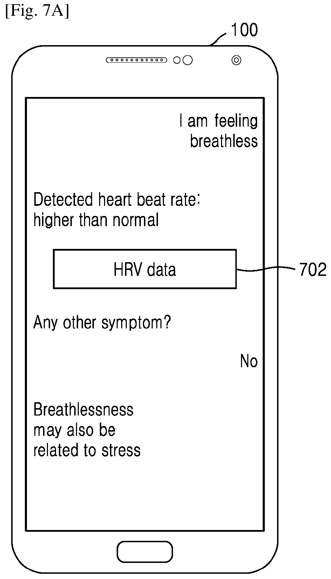

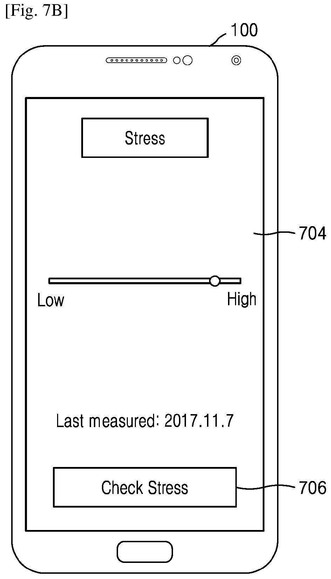

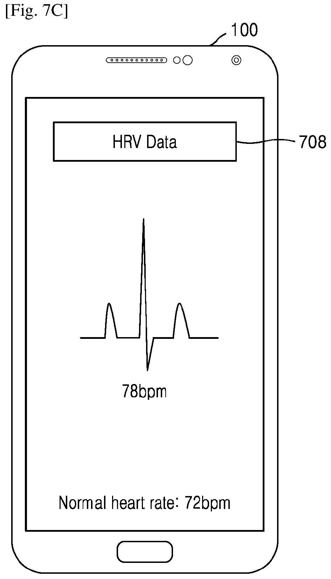

FIGS. 7A, 7B, and 7C are an example scenario illustrating implicit symptom context detection for identifying lifestyle related activities associated with symptoms received from the user, according to various embodiments of the disclosure.

Referring to FIG. 7A, in an embodiment, the electronic device 100 detects that data available in the medical record database 108 (e.g., medical information database) is not sufficient to provide a comprehensive analysis of the symptoms provided by the user and extra data needs to be acquired for analysis. Further, some of the data needs to be acquired in real time to provide better analysis of the health condition of the user. The electronic device 100 may acquire the data at a given instant of time by prompting the user to provide the data or by automatically detecting the required data by using multiple device sensors. The electronic device 100 may prompt the user to provide some data. For example, if the electronic device 100 has to record the pulse rate data of the user, the electronic device 100 may prompt the user to place a finger on a pulse rate sensor, and the like.

Breathlessness may be related to various chronic conditions like asthma, lung disorder, heart failure, etc. Further, breathlessness may also be due to a poor fitness level, an increased stress level, or an excessive workout, and may appear with other symptoms like coughing, chest pain, fever, etc. In an example scenario where the user provides the symptom to the electronic device 100 as 'breathlessness', the electronic device 100 commands the health application responsible for recording the heart rate of the user to record heart rate variability (HRV) of the user. HRV is a symptom used to detect a wide range of clinical conditions including problems related to the heart, mental issues like stress and anxiety, etc. The health application fetches the current heart rate variation of the user (which is not pre-stored in the medical record database 108), based on the context of the symptom provided by the user and provides HRV data in an HRV data widget 702, as shown in FIG. 7A. Further, the electronic device 100 analyses the heart rate variation of the user and concludes that the variation in the heart rate of the user could also be associated with a stress level of the user.

Referring to FIG. 7B, a stress related application 704 is used to provide details related to the stress level of the user. The stress related application 704 displays the stress level of the user and also provides a date on which the stress level was last measured (i.e., the previous day). Further, to obtain real time information related to the stress level of the user, the electronic device 100 prompts the user to select the 'check stress' option 706. The stress level of the user is measured implicitly without the intervention of the user, i.e., the user does not have to provide any input explicitly related to the stress level experienced.

Trend outlier detection follows a two step process where initially context is detected based on the symptom input by the user followed by detection of an event such as a variation of the heart rate which is then used to predict the cause of the symptom, i.e., the increased stress level of the user is detected based on the breathlessness symptom input by the user. Further, the event which led to the increased stress level of the user is detected to be the HRV and is identified as the cause of the breathlessness experienced by the user, as shown in FIG. 7C. In FIG. 7C, an HRV data widget 708 is shown with indicators.

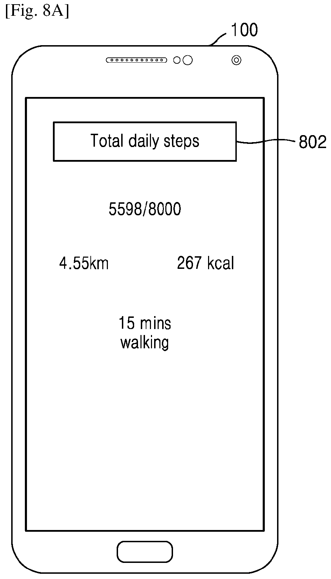

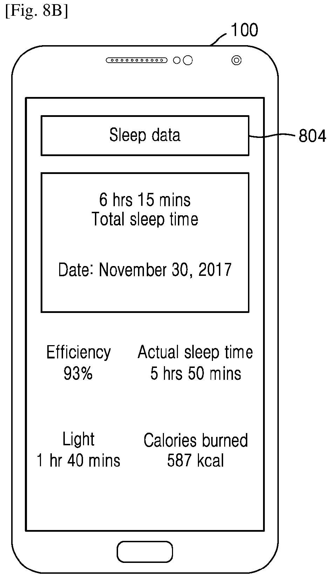

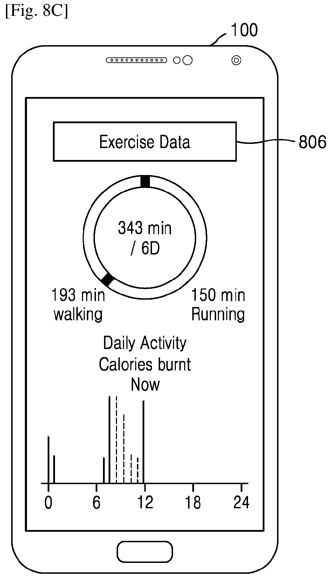

FIGS. 8A, 8B, and 8C are examples illustrating selection of a relevant health indicator for determining lifestyle related activities of a user associated with symptoms provided by the user, according to various embodiments of the disclosure.

In an embodiment, health indicators and activities of the user stored in the medical record database 108 may not be relevant in determining the lifestyle related activities associated with the symptoms provided by the user. Further, the electronic device 100 provides an activeness window for every health indicator and activity of the user stored in the medical record database 108. The health indicator and the activity of the user are valid only when the value of the health indicator and the activity of the user are within the activeness window. For example, information related to food consumed by the user two days previously may not be valid at the current time. However, a heavy workout session performed by the user two days previously may be valid at the current time. Hence, the activeness window varies for different health indicators and activities of the user and only contextually valid health indicators and activities are identified as being associated with the symptoms provided by the user.

Referring to FIG. 8A, in an example scenario where the user provides a symptom of sudden joint pain, the symptom may be related to an activity like excessive walking. Hence, health indicators such as a number of steps walked by the user become a valid health indicator to be considered in determining the cause of the joint pain symptom provided by the user. In FIG. 8A, a total daily steps widget 802 is shown with various indicators.

In another example scenario where the user provides a symptom of dizziness, the symptom may be related to sleep variation. Hence, activities such as sleep duration, wake up time, etc., becomes a valid activity to be considered in determining the cause of the dizziness experienced by the user, as shown in FIG. 8B. In FIG. 8B, a sleep data widget 804 is shown with various related indicators.

In another example scenario where the user provides a symptom which is body pain, the symptom may be related to a heavy activity such as an intensive workout session or running. Hence, activities such as the intensive workout session, running, etc., becomes a valid activity to be considered for determining the cause of the body pain experienced by the user, as shown in FIG. 8C. In FIG. 8C, an exercise data widget 806 is shown with various related indicators.

Generally, the correlation between chronic conditions, health indicators, and the symptoms are manually defined by medical practitioners and domain experts. The proposed disclosure develops a link between the chronic condition of the user, the health indicators, and the associated symptoms, as shown in Table 1.

**[Table 1]**

| **Chronic condition** | **Health indicator** | | **Symptom** |
|---|---|---|---|
| **Hyper Tension** | 1. Food Intake | ┐ | a) Heart Pain |
| | 2. Water Intake | ├ W1b | b) Excessive Sweating |
| | 3. Exercise | \| | c) Fatigue |
| | 4. Blood Pressure | ├ W1d | d) High HR |
| | 5. Sleep trends | ├ W1e | e) Dizziness |
| | 6. Oxygen trends | └ W1f | f) Nausea |
| | 7. Stress trends | | g) Difficulty in breathing |
| | 8. Inactivity | | h) Arm Pain |
| **Thyroid** | 1. Food Intake | | a) Throat Swelling |
| | 2. Water Intake | ┌ W3b | b) Fatigue |
| | 3. Exercise | ┤ | c) Weight Gain |
| | 4. Blood Pressure | ├ W3d | d) Weight Loss |
| | 5. Sleep trends | \| | e) Mood Swing |
| | 6. Oxygen trends | ├ W3f | f) Muscle Pain |
| | 7. Stress trends | └ W3f | g) Joint Pain |
| | 8. Body Temperature | | h) Dry Skin |
| | 9. Pulse Rate | | i) Hair Loss |

Further, each health indicator is provided a specific weight with respect to the symptoms and a correlation is defined for each of the health indicators considered. Hence, each health indicator is associated with a set of symptoms.

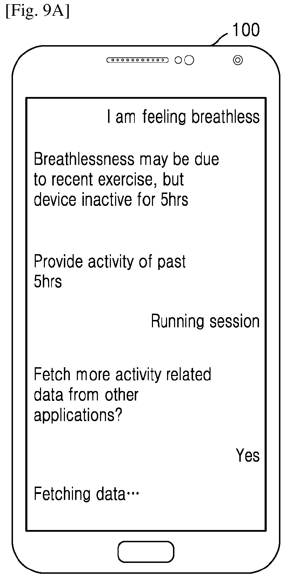

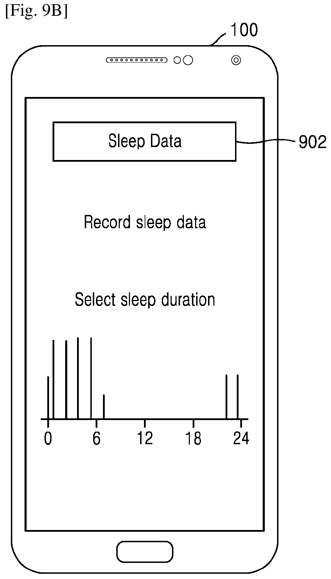

FIGS. 9A and 9B are example illustrations in which the electronic device 100 acquires relevant health indicators which are not stored in the medical record database 108, according to various embodiments of the disclosure.

In an embodiment, the health indicators and activities of the user may not be pre-stored at the medical record database 108 of the electronic device 100 and the electronic device 100 may either prompt the user to provide the missing data or the electronic device 100 may automatically detect the required data, in order to provide a comprehensive analysis of the symptoms provided by the user.

Referring to FIG. 9A, the electronic device 100 determines that the symptom (i.e., breathlessness) provided by the user has a high probability of occurrence due to a heavy activity health indicator. Therefore, even though the electronic device 100 was in an inactive state and data during the inactive state is missing, the electronic device 100 prompts the user to provide the missing information. Furthermore, analysis of the state of the electronic device 100 is performed based on user behavior (i.e., device usage analysis) to detect a possibility of unintentional lack of context while analyzing the symptoms provided by the user to detect the cause.

Referring to FIG. 9B, a sleep data application 902 is triggered to gather missing sleep related data when a sleep related health indicator is inactive and there is a high correlation between the sleep health indicator and the symptom provided by the user.

The example embodiments disclosed herein may be implemented using at least one software program running on at least one hardware device and performing network management functions to control the elements disclosed herein.

While the disclosure has been shown and described with reference to various embodiments thereof, it will be understood by those skilled in the art that various changes in form and details may be made therein without departing from the scope of the disclosure as defined by the appended claims

## Claims

1. A method of providing healthcare advice on a device (100) of a user, the method comprising:
receiving (202a), at the device (100), a user input indicating a symptom related to the user;
retrieving (204a), from the device (100), at least one medical information of the user;
determining the symptom related to the user being related to missing data during an inactive state of the device (100);
receiving a second user input corresponding to the missing data during the inactive state of the device;
detecting (206a) a user activity prior to the symptom arising by using at least one sensor (104);
correlating (208a) the user input indicating the symptom, the second user input corresponding to the missing data during the inactive state of the device, the at least one medical information of the user and the user activity prior to the symptom arising; and
displaying (210a) advice about the symptom based on the correlation.

2. The method of claim 1, wherein the detecting of the user activity prior to the symptom arising comprises:
detecting the user activity prior to the symptom arising by determining at least one of a movement, position in space, inspiratory time, expiratory time, elongation, motion, temperature, impact, speed, cadence, proximity, flexibility, movement, velocity, acceleration, posture, relative motion between limbs and trunk, location of the user using the at least one sensor.

3. The method of claim 1, further comprising:
detecting health indicators of the user,
wherein the correlating comprises correlating the user input, the missing data during the inactive state of the device, the at least one of medical information of the user, the user activity prior to the symptom arising, and the health indicators of the user.

4. The method of claim 1, further comprising:
in response to the user input indicating the symptom related to the user, displaying a query about one or more symptoms or at least one health indicator to the user;
receiving a third user input indicating the one or more symptoms or the at least one health indicator from the user, the third user input being composed of a natural language; and
analyzing the at least one health indicator, the user activity, the at least one medical information, or the symptom,
wherein the correlating is performed based on the analyzing.

5. The method of claim 4, further comprising:
storing the symptom, the at least one health indicators and the user activity in a database of the device (100) or in a database of a server.

6. The method of claim 1, further comprising:
identifying lifestyle parameters of the user;
wherein the correlating comprises correlating the user input indicating the symptom, the missing data during the inactive state of the device, the at least one of medical information of the user, the user activity prior to the symptom arising, and the lifestyle parameters of the user,
wherein the method further comprises identifying a cause of the symptom based on the correlation, and
wherein the advice comprises the cause of the symptom.

7. The method of claim 1, further comprising:
identifying the symptom based on parsing the user input using a natural language processing,
wherein the user input is composed of a natural language.

8. The method of claim 1, wherein the advice comprises one or more healthcare suggestions including a suggestion of a possible lifestyle related activity to mitigate the symptom or a suggestion of transmitting an alert to a caregiver of the user.

9. The method of claim 1, further comprising:
identifying the user activity as a cause of the symptom based on an analysis with and the correlating of the user input, the at least one medical information of the user and the user activity prior to the symptom arising,
wherein the displaying of the advice comprises displaying the advice including the cause of the symptom.

10. The method of claim 9, further comprising:
generating an electronic report including the symptoms and the cause of the symptom; and
transmitting the electronic report to a device of a caregiver of the user.

11. The method of claim 1, further comprising:
generating a data item representing at least one health indicators of the user, the data item launched in one or more health applications;
associating the symptom with the at least one health indicators, wherein the at least one health indicators is determined by an input on the data item by the user;
identifying at least one secondary symptom relevant to the at least one health indicators based on the association; and
updating the electronic report to include the at least one secondary symptom and a secondary cause of the at least one secondary symptom.

12. The method of claim 1, wherein the at least one medical information of the user comprises at least one of information input by the user, chronic condition of the user, information from medical reports of the user, consultation history, patient medication, information from one or more health applications of the user, and health indication parameters of the user.

13. An apparatus for providing healthcare advice, the apparatus comprising:
at least one sensor configured to detect a user activity;
at least one processor (118) configured to:
control a user interface to receive a user input indicating a symptom related to the user,
retrieve from a memory (116) at least one medical information of the user,
determine the symptom related to the user being related to missing data during an inactive state of the apparatus,
control the user interface to receive a second user input corresponding to the missing data during the inactive state of the apparatus,
correlate the user input indicating the symptom, the second user input corresponding to the missing data during the inactive state of the apparatus, the at least one medical information of the user and the user activity prior to the symptom arising, and
a display configured to display advice about the symptom based on the correlation,
wherein the at least one sensor is configured to detect the user activity prior to the symptom arising.

14. A non-transitory computer-readable storage medium storing instructions thereon that, when executed, cause at least one processor (118) to perform a method, the method comprising:
receiving (202a), at the device (100), a user input indicating a symptom related to the user;
retrieving (204a), from the device (100), at least one medical information of the user;
determining the symptom related to the user being related to missing data during an inactive state of the device;
receiving a second user input corresponding to the missing data during the inactive state of the device;
detecting (206a) a user activity prior to the symptom arising by using at least one sensor;
correlating (208a) the user input indicating the symptom, the second user input corresponding to the missing data during the inactive state of the device, the at least one medical information of the user and the user activity prior to the symptom arising; and
displaying (210a) advice about the symptom based on the correlation.

## Patentansprüche

1. Verfahren zur Bereitstellung von Gesundheitstipps auf einem Gerät (100) eines Benutzers, wobei das Verfahren Folgendes umfasst:
Empfangen (202a), auf dem Gerät (100), einer Benutzereingabe, die ein auf den Benutzer bezogenes Symptom angibt;
Abrufen (204a), aus dem Gerät (100), mindestens einer medizinischen Information des Benutzers;
Bestimmen, dass das auf den Benutzer bezogene Symptom sich auf fehlende Daten während eines inaktiven Zustands des Geräts (100) bezieht;
Empfangen einer zweiten Benutzereingabe, die den fehlenden Daten während des inaktiven Zustands des Geräts entspricht;
Detektieren (206a) einer Benutzeraktivität vor dem Auftreten des Symptoms durch Verwendung mindestens eines Sensors (104);
Korrelieren (208a) der das Symptom angebenden Benutzereingabe, wobei die zweite Benutzereingabe den fehlenden Daten während des inaktiven Zustands des Geräts, der mindestens einen medizinischen Information des Benutzers und der Benutzeraktivität vor dem Auftreten des Symptoms entspricht; und
Anzeigen (210a) von Tipps zu dem Symptom basierend auf der Korrelation.

2. Verfahren nach Anspruch 1, wobei das Detektieren der Benutzeraktivität vor dem Auftreten des Symptoms Folgendes umfasst:
Detektieren der Benutzeraktivität vor dem Auftreten des Symptoms durch Bestimmen mindestens eines von einer/einem Bewegung, Position im Raum, Inspirationszeit, Exspirationszeit, Streckung, Motion, Temperatur, Stoß, Geschwindigkeit, Trittfrequenz, Nähe, Flexibilität, Bewegung, Geschwindigkeit, Beschleunigung, Haltung, relativen Motion zwischen Gliedmaßen und Rumpf, Standort des Benutzers unter Verwendung des mindestens einen Sensors.

3. Verfahren nach Anspruch 1, ferner umfassend:
Detektieren von Gesundheitsindikatoren des Benutzers,
wobei das Korrelieren das Korrelieren der Benutzereingabe, der fehlenden Daten während des inaktiven Zustands des Geräts, der mindestens einen medizinischen Information des Benutzers, der Benutzeraktivität vor dem Auftreten des Symptoms und der Gesundheitsindikatoren des Benutzers umfasst.

4. Verfahren nach Anspruch 1, ferner umfassend:
als Reaktion auf die Benutzereingabe, die das auf den Benutzer bezogene Symptom angibt, Anzeigen einer Abfrage zu einem oder mehreren Symptomen oder mindestens einem Gesundheitsindikator an den Benutzer;
Empfangen einer dritten Benutzereingabe, die das eine oder die mehreren Symptome oder den mindestens einen Gesundheitsindikator von dem Benutzer angibt, wobei die dritte Benutzereingabe aus einer natürlichen Sprache besteht; und
Analysieren des mindestens einen Gesundheitsindikators, der Benutzeraktivität, der mindestens einen medizinischen Information oder des Symptoms,
wobei das Korrelieren basierend auf dem Analysieren durchgeführt wird.

5. Verfahren nach Anspruch 4, ferner umfassend:
Speichern des Symptoms, des mindestens einen Gesundheitsindikators und der Benutzeraktivität in einer Datenbank des Geräts (100) oder in einer Datenbank eines Servers.

6. Verfahren nach Anspruch 1, ferner umfassend:
Identifizieren von Lebensstilparametern des Benutzers;
wobei das Korrelieren das Korrelieren der das Symptom angebenden Benutzereingabe, der fehlenden Daten während des inaktiven Zustands des Geräts, der mindestens einen medizinischen Information des Benutzers, der Benutzeraktivität vor dem Auftreten des Symptoms und der Lebensstilparameter des Benutzers umfasst,
wobei das Verfahren ferner das Identifizieren einer Ursache des Symptoms basierend auf der Korrelation umfasst, und
wobei der Tipp die Ursache des Symptoms umfasst.

7. Verfahren nach Anspruch 1, ferner umfassend:
Identifizieren des Symptoms basierend auf dem Parsen der Benutzereingabe unter Verwendung einer natürlichen Sprachverarbeitung,
wobei die Benutzereingabe aus einer natürlichen Sprache besteht.

8. Verfahren nach Anspruch 1, wobei der Tipp einen oder mehrere Gesundheitsvorschläge umfasst, einschließlich eines Vorschlags einer möglichen lebensstilbezogenen Aktivität, um das Symptom abzumildern, oder eines Vorschlags des Sendens eines Alerts an eine Pflegekraft des Benutzers.

9. Verfahren nach Anspruch 1, ferner umfassend:
Identifizieren der Benutzeraktivität als eine Ursache des Symptoms basierend auf einer Analyse mit und dem Korrelieren der Benutzereingabe, der mindestens einen medizinischen Information des Benutzers und der Benutzeraktivität vor dem Auftreten des Symptoms,
wobei das Anzeigen des Tipps das Anzeigen des Tipps einschließlich der Ursache des Symptoms umfasst.

10. Verfahren nach Anspruch 9, ferner umfassend:
Erzeugen eines elektronischen Berichts einschließlich der Symptome und der Ursache des Symptoms; und
Senden des elektronischen Berichts an ein Gerät einer Pflegekraft des Benutzers.

11. Verfahren nach Anspruch 1, ferner umfassend:
Erzeugen eines Datenelements, das mindestens einen Gesundheitsindikator des Benutzers repräsentiert, wobei das Datenelement in einer oder mehreren Gesundheitsanwendungen gestartet wird;
Assoziieren des Symptoms mit dem mindestens einen Gesundheitsindikator, wobei der mindestens eine Gesundheitsindikator durch eine Eingabe zu dem Datenelement durch den Benutzer bestimmt wird;
Identifizieren mindestens eines sekundären Symptoms, das für den mindestens einen Gesundheitsindikator relevant ist, basierend auf der Assoziation; und
Aktualisieren des elektronischen Berichts, um das mindestens eine sekundäre Symptom und eine sekundäre Ursache des mindestens einen sekundären Symptoms einzuschließen.

12. Verfahren nach Anspruch 1, wobei die mindestens eine medizinische Information des Benutzers mindestens eines von Informationen, die von dem Benutzer eingegeben werden, chronischer Erkrankung des Benutzers, Informationen aus medizinischen Berichten des Benutzers, Konsultationsverlauf, Patientenmedikation, Informationen aus einer oder mehreren Gesundheitsanwendungen des Benutzers und Gesundheitsindikationsparametern des Benutzers umfasst.

13. Vorrichtung zum Bereitstellen von Gesundheitstipps, wobei die Vorrichtung Folgendes umfasst:
mindestens einen Sensor, der dazu konfiguriert ist, eine Benutzeraktivität zu detektieren;
mindestens einen Prozessor (118), der konfiguriert ist zum:
Steuern einer Benutzeroberfläche, um eine Benutzereingabe zu empfangen, die ein auf den Benutzer bezogenes Symptom angibt,
Abrufen, aus einem Speicher (116), mindestens einer medizinischen Information des Benutzers,
Bestimmen, ob das auf den Benutzer bezogene Symptom sich auf fehlende Daten während eines inaktiven Zustands der Vorrichtung bezieht,
Steuern der Benutzeroberfläche, um eine zweite Benutzereingabe zu empfangen, die den fehlenden Daten während des inaktiven Zustands der Vorrichtung entspricht,
Korrelieren der das Symptom angebenden Benutzereingabe, wobei die zweite Benutzereingabe den fehlenden Daten während des inaktiven Zustands der Vorrichtung, der mindestens einen medizinischen Information des Benutzers und der Benutzeraktivität vor dem Auftreten des Symptoms entspricht, und
eine Anzeige, die dazu konfiguriert ist, Tipps zu dem Symptom basierend auf der Korrelation anzuzeigen,
wobei der mindestens eine Sensor dazu konfiguriert ist, die Benutzeraktivität vor dem Auftreten des Symptoms zu detektieren.

14. Nicht-transitorisches computerlesbares Speichermedium, das Anweisungen darauf speichert, die, beim Ausführen, mindestens einen Prozessor (118) dazu veranlassen, ein Verfahren durchzuführen, wobei das Verfahren Folgendes umfasst:
Empfangen (202a), auf dem Gerät (100), einer Benutzereingabe, die ein auf den Benutzer bezogenes Symptom angibt;
Abrufen (204a), aus dem Gerät (100), mindestens einer medizinischen Information des Benutzers;
Bestimmen, dass das auf den Benutzer bezogene Symptom sich auf fehlende Daten während eines inaktiven Zustands des Geräts bezieht;
Empfangen einer zweiten Benutzereingabe, die den fehlenden Daten während des inaktiven Zustands des Geräts entspricht;
Detektieren (206a) einer Benutzeraktivität vor dem Auftreten des Symptoms durch Verwendung mindestens eines Sensors;
Korrelieren (208a) der das Symptom angebenden Benutzereingabe, wobei die zweite Benutzereingabe den fehlenden Daten während des inaktiven Zustands des Geräts, der mindestens einen medizinischen Information des Benutzers und der Benutzeraktivität vor dem Auftreten des Symptoms entspricht; und
Anzeigen (210a) von Tipps zu dem Symptom basierend auf der Korrelation.

## Revendications

1. Procédé de fourniture de conseil en soins de santé sur un dispositif (100) d'un utilisateur, le procédé comprenant :
la réception (202a), au niveau du dispositif (100), d'une entrée utilisateur indiquant un symptôme associé à l'utilisateur ;
l'extraction (204a), à partir du dispositif (100), d'au moins une information médicale de l'utilisateur ;
la détermination du fait que le symptôme associé à l'utilisateur est associé à des données manquantes pendant un état inactif du dispositif (100) ;
la réception d'une deuxième entrée utilisateur correspondant aux données manquantes pendant l'état inactif du dispositif ;
la détection (206a) d'une activité de l'utilisateur avant l'apparition du symptôme à l'aide d'au moins un capteur (104) ;
la mise en corrélation (208a) de l'entrée utilisateur indiquant le symptôme, de la deuxième entrée utilisateur correspondant aux données manquantes pendant l'état inactif du dispositif, de ladite au moins une information médicale de l'utilisateur et de l'activité de l'utilisateur avant l'apparition du symptôme ; et
l'affichage (210a) d'un conseil au sujet du symptôme sur la base de la mise en corrélation.

2. Procédé selon la revendication 1, ladite détection de l'activité de l'utilisateur avant l'apparition du symptôme comprenant :
la détection de l'activité de l'utilisateur avant l'apparition du symptôme en déterminant au moins un élément parmi un mouvement, la position dans l'espace, la durée d'inspiration, la durée d'expiration, une élongation, un déplacement, la température, un choc, la vitesse, la cadence, la proximité, la flexibilité, un mouvement, la vélocité, l'accélération, une posture, un mouvement relatif entre les membres et le tronc, l'emplacement de l'utilisateur à l'aide dudit au moins un capteur.

3. Procédé selon la revendication 1, comprenant en outre :
la détection des indicateurs de santé de l'utilisateur,
ladite mise en corrélation comprenant la mise en corrélation de l'entrée utilisateur, des données manquantes pendant l'état inactif du dispositif, de ladite au moins l'une des informations médicales de l'utilisateur, de l'activité de l'utilisateur avant l'apparition du symptôme et des indicateurs de santé de l'utilisateur.

4. Procédé selon la revendication 1, comprenant en outre :
en réponse à l'entrée utilisateur indiquant le symptôme associé à l'utilisateur, l'affichage d'une requête concernant un ou plusieurs symptômes ou au moins un indicateur de santé à l'utilisateur ;
la réception d'une troisième entrée utilisateur indiquant lesdits un ou plusieurs symptômes ou ledit au moins un indicateur de santé de l'utilisateur, la troisième entrée utilisateur étant composée d'un langage naturel ; et
l'analyse dudit au moins un indicateur de santé, de l'activité de l'utilisateur, de ladite au moins une information médicale ou du symptôme,
ladite mise en corrélation étant réalisée sur la base de l'analyse.

5. Procédé selon la revendication 4, comprenant en outre :
le stockage du symptôme, dudit au moins un indicateur de santé et de l'activité de l'utilisateur dans une base de données du dispositif (100) ou dans une base de données d'un serveur.

6. Procédé selon la revendication 1, comprenant en outre :
l'identification de paramètres de style de vie de l'utilisateur ;
ladite mise en corrélation comprenant la mise en corrélation de l'entrée utilisateur indiquant le symptôme, des données manquantes pendant l'état inactif du dispositif, de ladite au moins l'une des informations médicales de l'utilisateur, de l'activité de l'utilisateur avant l'apparition du symptôme et des paramètres de mode de vie de l'utilisateur,
ledit procédé comprenant en outre l'identification d'une cause du symptôme sur la base de la mise en corrélation, et
ledit conseil comprenant la cause du symptôme.

7. Procédé selon la revendication 1, comprenant en outre :
l'identification du symptôme sur la base du parsage de l'entrée utilisateur à l'aide d'un traitement de langage naturel,
ladite entrée utilisateur étant composée d'un langage naturel.

8. Procédé selon la revendication 1, ledit conseil comprenant une ou plusieurs suggestions de soins de santé comprenant une suggestion d'une activité possible associée au mode de vie pour atténuer le symptôme ou une suggestion de transmission d'une alerte à un soignant de l'utilisateur.

9. Procédé selon la revendication 1, comprenant en outre :
l'identification de l'activité de l'utilisateur en tant que cause du symptôme sur la base d'une analyse avec l'entrée utilisateur et de la mise en corrélation de celle-ci, de ladite au moins une information médicale de l'utilisateur et de l'activité de l'utilisateur avant l'apparition du symptôme,
ledit affichage du conseil comprenant l'affichage du conseil comprenant la cause du symptôme.

10. Procédé selon la revendication 9, comprenant en outre :
la génération d'un rapport électronique comprenant les symptômes et la cause du symptôme ; et
la transmission du rapport électronique à un dispositif d'un soignant de l'utilisateur.

11. Procédé selon la revendication 1, comprenant en outre :
la génération d'un élément de donnée représentant au moins un indicateur de santé de l'utilisateur, l'élément de donnée étant lancée dans une ou plusieurs applications de santé ;
l'association du symptôme avec ledit au moins un indicateur de santé, ledit au moins un indicateur de santé étant déterminé par une entrée sur l'élément de donnée par l'utilisateur ;
l'identification d'au moins un symptôme secondaire pertinent pour ledit au moins un indicateur de santé sur la base de l'association ; et
la mise à jour du rapport électronique pour inclure ledit au moins un symptôme secondaire et une cause secondaire dudit au moins un symptôme secondaire.

12. Procédé selon la revendication 1, ladite au moins une information médicale de l'utilisateur comprenant au moins l'une des informations entrées par l'utilisateur, l'état chronique de l'utilisateur, des informations provenant des rapports médicaux de l'utilisateur, l'historique des consultations, les médicaments du patient, les informations provenant d'une ou plusieurs applications de santé de l'utilisateur et des paramètres d'indication de santé de l'utilisateur.

13. Appareil permettant la fourniture d'un conseil en soins de santé, l'appareil comprenant :
au moins un capteur configuré pour détecter une activité de l'utilisateur ;
au moins un processeur (118) configuré pour :
commander à une interface utilisateur de recevoir une entrée utilisateur indiquant un symptôme associé à l'utilisateur,
récupérer à partir d'une mémoire (116) au moins une information médicale de l'utilisateur,
déterminer le fait que le symptôme associé à l'utilisateur est associé à des données manquantes pendant un état inactif de l'appareil,
commander à l'interface utilisateur de recevoir une deuxième entrée utilisateur correspondant aux données manquantes pendant l'état inactif de l'appareil,
mettre en corrélation l'entrée utilisateur indiquant le symptôme, la deuxième entrée utilisateur correspondant aux données manquantes pendant l'état inactif de l'appareil, ladite au moins une information médicale de l'utilisateur et l'activité de l'utilisateur avant l'apparition du symptôme, et
un dispositif d'affichage configuré pour afficher un conseil au sujet du symptôme sur la base de la mise en corrélation,
ledit au moins un capteur étant configuré pour détecter l'activité de l'utilisateur avant l'apparition du symptôme.

14. Support de stockage non transitoire lisible par ordinateur sur lequel sont stockées des instructions qui, lorsqu'elles sont exécutées, amènent au moins un processeur (118) à exécuter un procédé, le procédé comprenant :
la réception (202a), au niveau du dispositif (100), d'une entrée utilisateur indiquant un symptôme associé à l'utilisateur ;
la récupération (204a), à partir du dispositif (100), d'au moins une information médicale de l'utilisateur ;
la détermination du fait que le symptôme associé à l'utilisateur est associé à des données manquantes pendant un état inactif du dispositif ;
la réception d'une deuxième entrée utilisateur correspondant aux données manquantes pendant l'état inactif du dispositif ;
la détection (206a) d'une activité de l'utilisateur avant l'apparition du symptôme à l'aide d'au moins un capteur ;
la mise en corrélation (208a) de **l'entrée** utilisateur indiquant le symptôme, de la deuxième entrée utilisateur correspondant aux données manquantes pendant l'état inactif du dispositif, de ladite au moins une information médicale de l'utilisateur et de l'activité de l'utilisateur avant l'apparition du symptôme ; et
l'affichage (210a) d'un conseil au sujet du symptôme sur la base de la mise en corrélation.
